# EUROPEAN PATENT APPLICATION

(11) **EP 4 454 826 A1**
(43) Date of publication of application: **30.10.2024**
(21) Application number: 22911100.0
(22) Date of filing: 16.12.2022
(51) Int. Cl.: B25J 3/00, A61B 34/32

(54) **SURGERY ASSISTANCE SYSTEM, SURGERY ASSISTANCE ROBOT, AND CONTROL METHOD FOR SURGERY ASSISTANCE SYSTEM**

(30) Priority: 22.12.2021 JP 2021208660
(71) Applicant: KAWASAKI JUKOGYO KABUSHIKI KAISHA, Kobe-shi, Hyogo 650-8670 (JP)
(72) Inventor: YASUDA, Tatsurou, Hyogo 650-8670 (JP)
(74) Representative: Hoffmann Eitle
(86) International application number: PCT/JP2022/046335
(87) International publication number: WO 2023/120396

(57) **Abstract**

A robotic surgical system (100) includes a control device (130) configured or programmed to detect a position and orientation of a trocar (T) based on a detection result of a detector (51, 52), and move a surgical instrument mount (73) toward the trocar (T) based on the detected position and orientation of the trocar (T).

## Description

### Technical Field

The present disclosure relates to a robotic surgical system, a surgical robot, and a control method for a robotic surgical system.

### Background Art

Conventionally, a robotic surgical system including a robot arm to which a surgical instrument is attached is known. For example, Japanese Translation of PCT International Application Publication No. 2017-515522 discloses a teleoperational assembly including a plurality of robot arms to which surgical instruments are attached, and a touchpad. In Japanese Translation of PCT International Application Publication No. 2017-515522, a reference laser line is radiated from the teleoperational assembly. An operator moving the teleoperational assembly follows a guided setup screen prompt "Push all arms behind the green laser line" shown on a touchscreen monitor and a voice prompt to push and move four robot arms such that the plurality of robot arms are arranged along the reference laser line. Thus, each robot arm to which the surgical instrument is attached is placed at an appropriate position.

### Prior Art

### Patent Document

Patent Document 1: Japanese Translation of PCT International Application Publication No. 2017-515522

### Summary of the Invention

However, in Japanese Translation of PCT International Application Publication No. 2017-515522, the operator needs to push and move each of the four robot arms in order to place, at the appropriate positions, the four robot arms to which the surgical instruments are attached. Therefore, the workload on the operator increases.

The present disclosure is intended to solve the above problem. The present disclosure aims to provide a robotic surgical system, a surgical robot, and a control method for a robotic surgical system each capable of reducing the workload on an operator to place a robot arm at an appropriate position.

In order to attain the aforementioned object, a robotic surgical system according to a first aspect of the present disclosure includes a robot arm including a surgical instrument mount to which a surgical instrument is attached, a detector to detect a position and orientation of a trocar inserted into a patient, and a control device. The control device is configured or programmed to detect the position and orientation of the trocar based on a detection result of the detector, and move the surgical instrument mount toward the trocar based on the detected position and orientation of the trocar.

In the robotic surgical system according to the first aspect of the present disclosure, as described above, the control device is configured or programmed to detect the position and orientation of the trocar based on the detection result of the detector, and move the surgical instrument mount toward the trocar based on the detected position and orientation of the trocar. Accordingly, the surgical instrument mount of the robot arm is automatically moved to an appropriate position based on the detected position and orientation of the trocar, and thus an operator does not need to manually move the surgical instrument mount of the robot arm. Therefore, the workload on the operator to place the robot arm at the appropriate position can be reduced. Furthermore, the robot arm is automatically placed at the appropriate position, and thus the time required to place the robot arm at the appropriate position can be reduced as compared with a case in which the robot arm is manually placed at the appropriate position.

A surgical robot according to a second aspect of the present disclosure includes a robot arm including a surgical instrument mount to which a surgical instrument is attached, a detector to detect a position and orientation of a trocar inserted into a patient, and a control device. The control device is configured or programmed to detect the position and orientation of the trocar based on a detection result of the detector, and move the surgical instrument mount toward the trocar based on the detected position and orientation of the trocar.

In the surgical robot according to the second aspect of the present disclosure, as described above, the control device is configured or programmed to detect the position and orientation of the trocar based on the detection result of the detector, and move the surgical instrument mount toward the trocar based on the detected position and orientation of the trocar. Accordingly, the surgical instrument mount of the robot arm is automatically moved to an appropriate position based on the detected position and orientation of the trocar, and thus an operator does not need to manually move the surgical instrument mount of the robot arm. Therefore, it is possible to provide the surgical robot capable of reducing the workload on the operator to place the robot arm at the appropriate position. Furthermore, the robot arm is automatically placed at the appropriate position, and thus it is possible to provide the surgical robot capable of reducing the time required to place the robot arm at the appropriate position as compared with a case in which the robot arm is manually placed at the appropriate position.

A control method for a robotic surgical system according to a third aspect of the present disclosure is a control method for a robotic surgical system including a robot arm including a surgical instrument mount to which a surgical instrument is attached, a detector to detect a position and orientation of a trocar inserted into a patient, and a control device, and includes detecting the position and orientation of the trocar based on a detection result of the detector, and moving the surgical instrument mount toward the trocar based on the detected position and orientation of the trocar.

As described above, the control method for the robotic surgical system according to the third aspect of the present disclosure includes moving the surgical instrument mount toward the trocar based on the detected position and orientation of the trocar. Accordingly, the surgical instrument mount of the robot arm is automatically moved to an appropriate position based on the detected position and orientation of the trocar, and thus an operator does not need to manually move the surgical instrument mount of the robot arm. Therefore, it is possible to provide the control method for the robotic surgical system capable of reducing the workload on the operator to place the robot arm at the appropriate position. Furthermore, the robot arm is automatically placed at the appropriate position, and thus it is possible to provide the control method for the robotic surgical system capable of reducing the time required to place the robot arm at the appropriate position as compared with a case in which the robot arm is manually placed at the appropriate position.

According to the present disclosure, as described above, it is possible to reduce the workload on the operator to place the robot arm at the appropriate position.

### Brief Description of the Drawings

FIG. 1 is a diagram showing the configuration of a robotic surgical system according to an embodiment.
FIG. 2 is a diagram showing a display of a medical cart according to the embodiment.
FIG. 3 is a diagram showing the configuration of the medical cart according to the embodiment.
FIG. 4 is a diagram showing the configuration of a robot arm according to the embodiment.
FIG. 5 is a diagram showing a pair of forceps.
FIG. 6 is a perspective view showing the configuration of an arm operation unit according to the embodiment.
FIG. 7 is a diagram for illustrating translational movement of the robot arm.
FIG. 8 is a diagram for illustrating rotational movement of the robot arm.
FIG. 9 is a control block diagram of a surgical robot according to the embodiment.
FIG. 10 is a control block diagram of the robot arm according to the embodiment.
FIG. 11 is a control block diagram of the medical cart and a positioner according to the embodiment.
FIG. 12 is a flowchart for illustrating a control method for the surgical robot according to the embodiment.
FIG. 13 is a diagram showing a display of the medical cart according to the embodiment when a surgical site is set.
FIG. 14 is a diagram showing the display of the medical cart according to the embodiment when the medical cart approaches a patient.
FIG. 15 is a diagram showing a state in which an imager is imaging the patient.
FIG. 16 is a diagram showing a state before alignment between trocars and marks displayed on the display.
FIG. 17 is a diagram showing a state after alignment between the trocars and the marks displayed on the display.
FIG. 18 is a diagram showing an endoscope.
FIG. 19 is a diagram showing a pivot position setting instrument.
FIG. 20 is a diagram showing a state in which a second link has been moved to the vicinity of a trocar.
FIG. 21 is a diagram for illustrating operation to store a pivot position in a storage.

### Modes for Carrying Out the Invention

### Configuration of Robotic Surgical System

The configuration of a robotic surgical system 100 according to the present embodiment is now described. The robotic surgical system 100 includes a surgical robot 1 and a remote control apparatus 2.

In this description, the longitudinal direction of a surgical instrument 4 is defined as a Z direction. The distal end side of the surgical instrument 4 is defined as a Z1 side, and the proximal end side of the surgical instrument 4 is defined as a Z2 side. A direction perpendicular to the Z direction is defined as an X direction. One side in the X direction is defined as an X1 side, and the other side in the X direction is defined as an X2 side. A direction perpendicular to the Z direction and the X direction is defined as a Y direction. One side in the Y direction is defined as a Y1 side, and the other side in the Y direction is defined as a Y2 side.

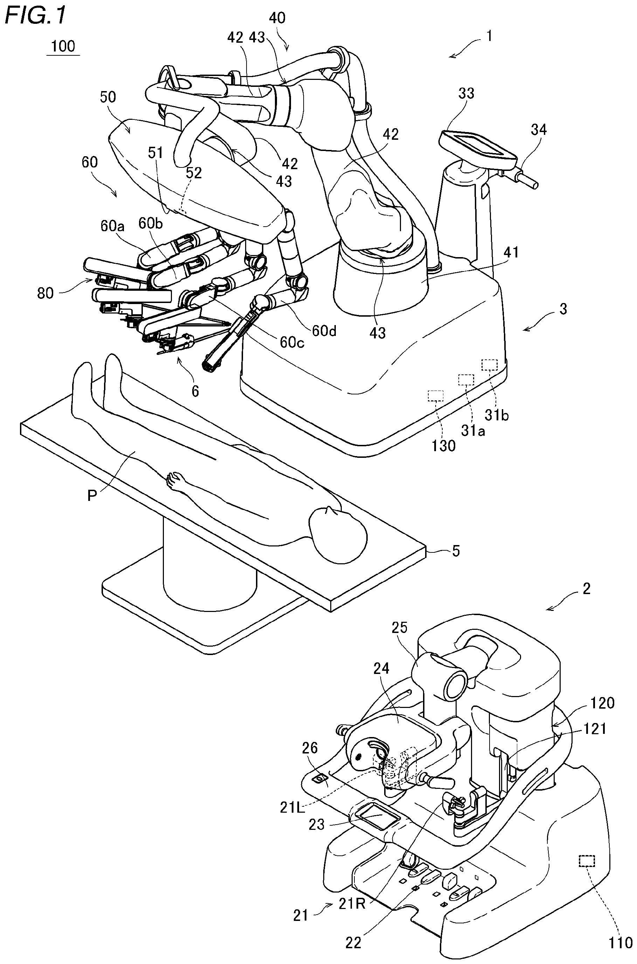

As shown in FIG. 1, the surgical robot 1 is arranged in an operating room. The remote control apparatus 2 is spaced apart from the surgical robot 1. An operator such as a doctor inputs a command to the remote control apparatus 2 to cause the surgical robot 1 to perform a desired operation. The remote control apparatus 2 transmits the input command to the surgical robot 1. The surgical robot 1 operates based on the received command. The surgical robot 1 is arranged in the operating room that is a sterilized sterile field.

### Configuration of Surgical Robot

As shown in FIG. 1, the surgical robot 1 includes a medical cart 3, a positioner 40, an arm base 50, an imager 51, a distance sensor 52, a plurality of robot arms 60, and arm operation units 80. The imager 51 and the distance sensor 52 are examples of a detector.

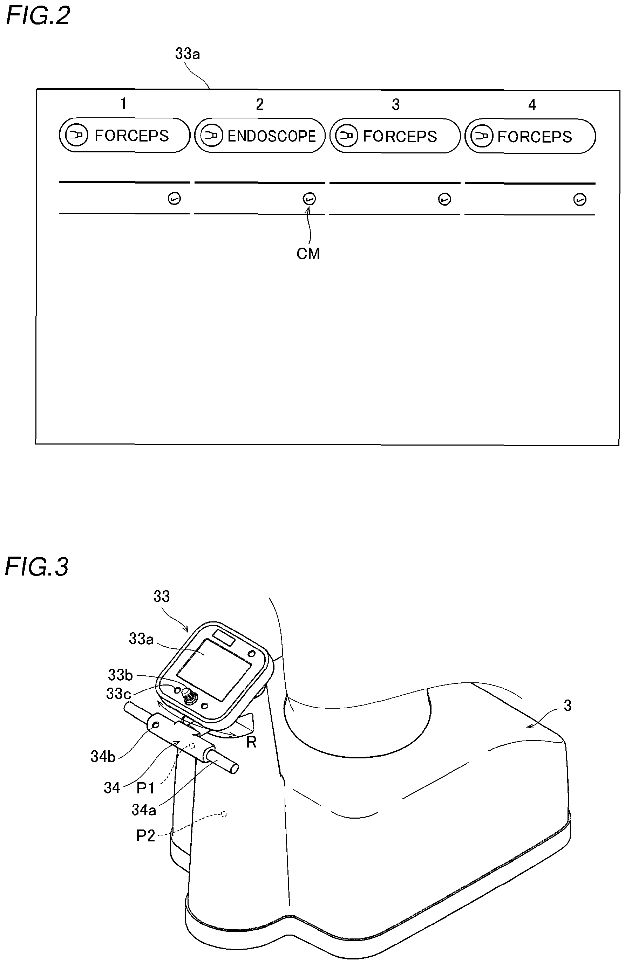

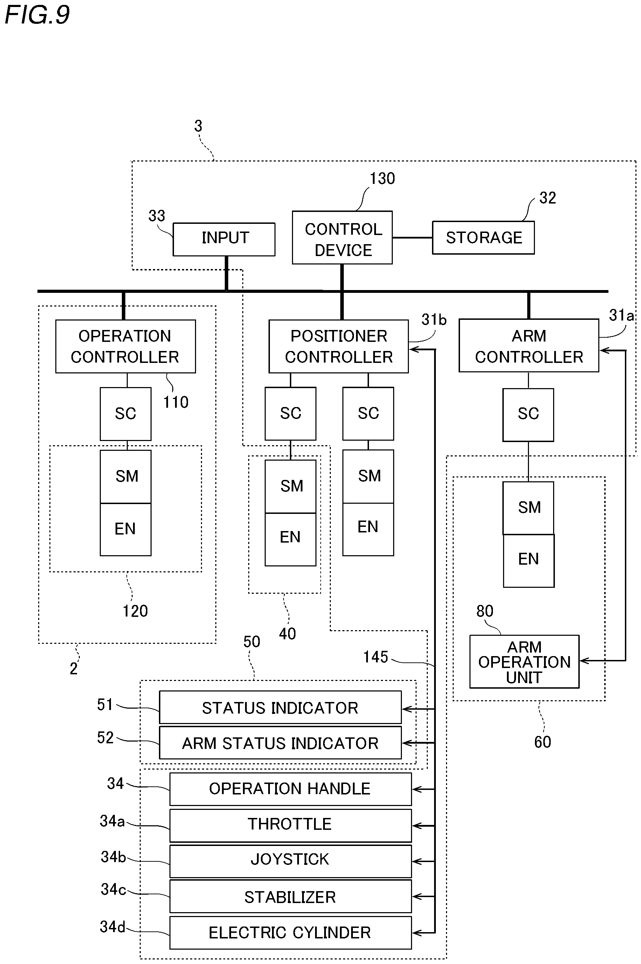

As shown in FIG. 3, the medical cart 3 moves the positioner 40. The medical cart 3 includes an input 33. The input 33 receives operations to move the positioner 40, the arm base 50, and the plurality of robot arms 60 or change their postures mainly in order to prepare for surgery before the surgery. The medical cart 3 includes an operation handle 34, and a stabilizer 34c and an electric cylinder 34d that are shown in FIG. 9.

As shown in FIG. 2, the input 33 includes a display 33a. The display 33a is a liquid crystal panel, for example. Numbers corresponding to the plurality of robot arms 60 are displayed on the display 33a. Furthermore, the type of surgical instrument 4 attached to each of the plurality of robot arms 60 is displayed on the display 33a. A check mark CM indicating that a pivot position PP described below has been set is displayed on the display 33a.

As shown in FIG 3, a joystick 33b for operating movement of the positioner 40 is arranged in the vicinity of the input 33 of the medical cart 3. The positioner 40 is three-dimensionally moved by selecting an operation mode displayed on the input 33 and operating the joystick 33b.

In the vicinity of the joystick 33b of the medical cart 3, an enable switch 33c is provided to enable or disable movement of the positioner 40. The joystick 33b is operated while the enable switch 33c is being pressed to enable movement of the positioner 40 such that the positioner 40 is moved.

The operation handle 34 is arranged in the vicinity of the display 33a of the medical cart 3. The operation handle 34 includes a throttle 34a that is gripped and twisted by an operator such as a nurse or a technician to operate movement of the medical cart 3. Specifically, the operation handle 34 is arranged below the input 33. As the throttle 34a is twisted from the near side to the far side, the medical cart 3 moves forward. As the throttle 34a is twisted from the far side to the near side, the medical cart 3 moves rearward. The speed of the medical cart 3 is changed according to a twisting amount of the throttle 34a. The operation handle 34 is rotatable to the left and right shown by an R direction, and the medical cart 3 is turned with rotation of the operation handle 34.

An enable switch 34b for enabling or disabling movement of the medical cart 3 is provided on the operation handle 34 of the medical cart 3. When the throttle 34a of the operation handle 34 is operated while the enable switch 34b is being pressed to enable movement of the medical cart 3, the medical cart 3 is moved.

As shown in FIG. 1, the positioner 40 includes a 7-axis articulated robot, for example. The positioner 40 is arranged on the medical cart 3. The positioner 40 adjusts the position of the arm base 50. The positioner 40 moves the position of the arm base 50 three-dimensionally.

The positioner 40 includes a base 41 and a plurality of links 42 coupled to the base 41. The plurality of links 42 are coupled to each other by joints 43.

The arm base 50 is attached to a distal end of the positioner 40. A proximal end of each of the plurality of robot arms 60 is attached to the arm base 50. Each of the plurality of robot arms 60 is able to take a folded and stored posture. The arm base 50 and the plurality of robot arms 60 are covered with sterile drapes and used. Moreover, each of the robot arms 60 supports the surgical instrument 4.

In this embodiment, the imager 51 and the distance sensor 52 are arranged on the arm base 50. The imager 51 and the distance sensor 52 detect the position and orientation of a trocar T inserted into a patient P. The imager 51 is a monocular camera. The imager 51 images the patient P. The distance sensor 52 is arranged adjacent to the imager 51 on the arm base 50. The distance sensor 52 is a TOF sensor, for example. The trocar T is not a port that is a hole created in the patient P, but a medical instrument inserted into the port. The trocar T is sometimes called a cannula.

A status indicator 53 and an arm status indicator 54 that are shown in FIG. 9 are provided on the arm base 50. The status indicator 53 indicates the status of the robotic surgical system 100. The arm status indicator 54 indicates the statuses of the robot arms 60.

The plurality of robot arms 60 are arranged. Specifically, four robot arms 60a, 60b, 60c, and 60d are arranged. The robot arms 60a, 60b, 60c, and 60d have the same or similar configurations as each other.

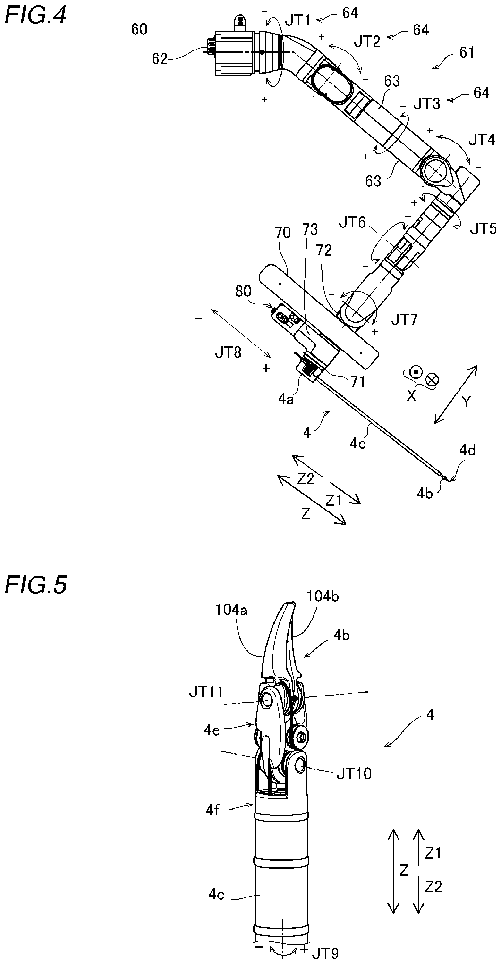

As shown in FIG. 4, each robot arm 60 includes an arm portion 61, a first link 72, a second link 73, and a translation mechanism 70. The robot arm 60 includes a JT1 axis, a JT2 axis, a JT3 axis, a JT4 axis, a JT5 axis, a JT6 axis, and a JT7 axis as rotation axes and a JT8 axis as a linear motion axis. The JT1 to JT7 axes are rotation axes of joints 64 of the arm portion 61. Furthermore, the JT7 axis is a rotation axis of the first link 72. The JT8 axis is a linear motion axis along which the translation mechanism 70 moves the second link 73 relative to the first link 72 along the Z direction. The second link 73 is an example of a surgical instrument mount.

The arm portion 61 includes a 7-axis articulated robot arm. The first link 72 is arranged at a distal end of the arm portion 61. An arm operation unit 80 described below is attached to the second link 73. The translation mechanism 70 is arranged between the first link 72 and the second link 73. A holder 71 that holds the surgical instrument 4 is arranged on the second link 73.

The surgical instrument 4 is attached to a distal end of each of the plurality of robot arms 60. The surgical instrument 4 includes a replaceable instrument, an endoscope 6 to capture an image of a surgical site, or a pivot position setting instrument 7 to set the pivot position PP described below, for example. The surgical instrument 4 as the instrument includes a driven unit 4a, a pair of forceps 4b, and a shaft 4c. The endoscope 6 and the pivot position setting instrument 7 are examples of a surgical instrument including a shaft.

As shown in FIG. 1, the endoscope 6 is attached to the distal end of one of the plurality of robot arms 60, such as the robot arm 60c, and surgical instruments 4 other than the endoscope 6 are attached to the distal ends of the remaining robot arms 60a, 60b, and 60d, for example. The endoscope 6 is attached to one of two robot arms 60b and 60c arranged in the center among the four robot arms 60 arranged adjacent to each other. The robot arms 60a, 60b, and 60d are examples of a first robot arm. The robot arm 60c is an example of a second robot arm.

### Configuration of Instrument

As shown in FIG. 5, the pair of forceps 4b is provided at a distal end of the instrument, for example. At the distal end of the instrument, in addition to the pair of forceps 4b, a pair of scissors, a grasper, a needle holder, a microdissector, a stable applier, a tacker, a suction cleaning tool, a snare wire, a clip applier, etc. are arranged as instruments having joints. At the distal end of the instrument, a cutting blade, a cautery probe, a washer, a catheter, a suction orifice, etc. are arranged as instruments having no joint.

The pair of forceps 4b includes a first support 4e and a second support 4f. The first support 4e supports the proximal end sides of jaw members 104a and 104b such that the proximal end sides of the jaw members 104a and 104b are rotatable about a JT11 axis. The second support 4f supports the proximal end side of the first support 4e such that the proximal end side of the first support 4e is rotatable about a JT10 axis. The shaft 4c rotates about a JT9 axis. The jaw members 104a and 104b pivot about the JT11 axis to open and close.

### Configuration of Arm Operation Unit

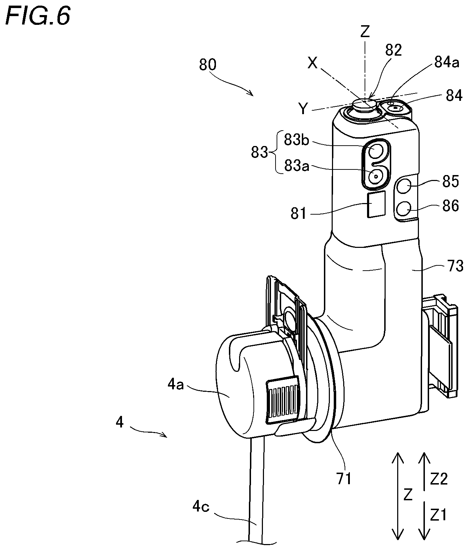

As shown in FIG. 6, the arm operation unit 80 is attached to the robot arm 60 to operate the robot arm 60. Specifically, the arm operation unit 80 is attached to the second link 73.

The arm operation unit 80 includes an enable switch 81, a joystick 82, and linear switches 83, a mode switching button 84, a mode indicator 84a, a pivot button 85, and an adjustment button 86.

The enable switch 81 enables or disables movement of the robot arm 60 in response to the joystick 82 and the linear switches 83. When the enable switch 81 is pressed by an operator such as a nurse or an assistant grasping the arm operation unit 80, movement of the surgical instrument 4 by the robot arm 60 is enabled.

The joystick 82 is an operation tool to control movement of the surgical instrument 4 by the robot arm 60. The joystick 82 controls a moving direction and a moving speed of the robot arm 60. The robot arm 60 is moved in accordance with a tilting direction and a tilting angle of the joystick 82.

The linear switches 83 are switches to move the surgical instrument 4 in the Z direction, which is the longitudinal direction of the surgical instrument 4. The linear switches 83 include a linear switch 83a to move the surgical instrument 4 in a direction in which the surgical instrument 4 is inserted into the patient P, and a linear switch 83b to move the surgical instrument 4 in a direction in which the surgical instrument 4 is moved away from the patient P. Both the linear switch 83a and the linear switch 83b are push-button switches.

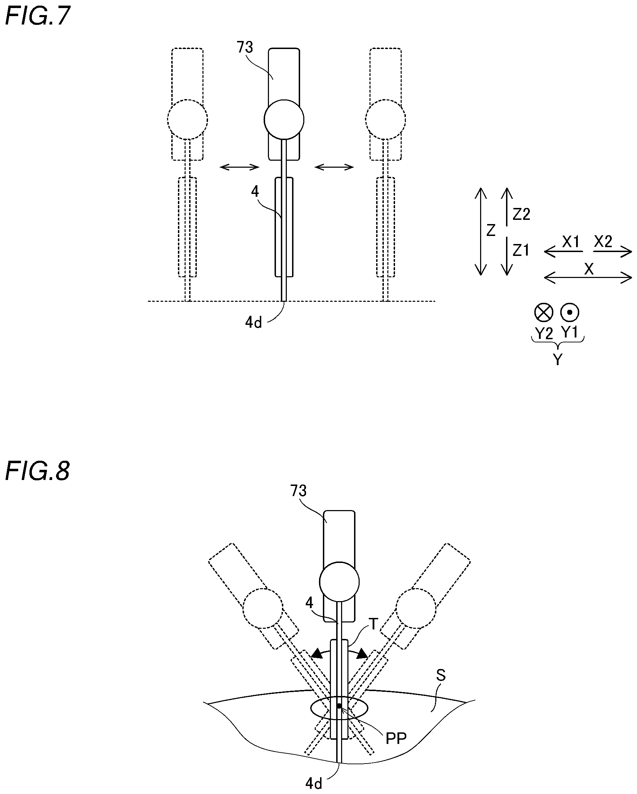

The mode switching button 84 is a push-button switch to switch between a mode for translationally moving the surgical instrument 4 as shown in FIG. 7 and a mode for rotationally moving the surgical instrument 4 as shown in FIG. 8. As shown in FIG. 7, in the mode for translationally moving the robot arm 60, the robot arm 60 is moved such that a distal end 4d of the surgical instrument 4 is moved in an X-Y plane. As shown in FIG. 8, in the mode for rotationally moving the robot arm 60, the robot arm 60 is moved such that the surgical instrument 4 is rotationally moved about a center of the JT11 axis of the pair of forceps 4b as a fulcrum when any pivot position PP is not stored in a storage 32, and the surgical instrument 4 is rotationally moved about the pivot position PP as a fulcrum when the pivot position PP is stored in the storage 32. In this case, the surgical instrument 4 is rotationally moved with the shaft 4c of the surgical instrument 4 inserted into the trocar T. The mode switching button 84 is arranged on a Z-direction side surface of the arm operation unit 80.

The mode indicator 84a indicates a switched mode. The mode indicator 84a is on to indicate a rotational movement mode and is off to indicate a translational movement mode. Furthermore, the mode indicator 84a also serves as a pivot position indicator that indicates that the pivot position PP has been set. The mode indicator 84a is arranged on the Z-direction side surface of the arm operation unit 80.

The pivot button 85 is a push-button switch to set the pivot position PP that serves as a fulcrum for movement of the surgical instrument 4 attached to the robot arm 60.

The adjustment button 86 is a button to optimize the position of the robot arm 60. After the pivot position PP for the robot arm 60 to which the endoscope 6 has been attached is set, the positions of the other robot arms 60 and the arm base 50 are optimized when the adjustment button 86 is pressed.

### Remote Control Apparatus

As shown in FIG. 1, the remote control apparatus 2 is arranged inside or outside the operating room, for example. The remote control apparatus 2 includes an operation unit 120 including arms 121 and an operation handle 21, foot pedals 22, a touch panel 23, a monitor 24, a support arm 25, and a support bar 26. The operation unit 120 includes an operation handle for the operator such as a doctor to input a command.

The operation unit 120 includes a handle to operate the surgical instrument 4. The operation unit 120 receives an operation amount for the surgical instrument 4. The operation unit 120 includes an operation unit 120L located on the left side as viewed from the operator such as a doctor and operated by the left hand of the operator, and an operation unit 120R located on the right side and operated by the right hand of the operator. The operation unit 120L and the operation unit 120R include an operation handle 21L and an operation handle 21R, respectively.

The monitor 24 is a scope-type display that displays an image captured by the endoscope 6. The support arm 25 supports the monitor 24 so as to align the height of the monitor 24 with the height of the face of the operator such as a doctor. The touch panel 23 is arranged on the support bar 26. The head of the operator is detected by a sensor provided in the vicinity of the monitor 24 such that the surgical robot 1 can be operated by the remote control apparatus 2. The operator operates the operation unit 120 and the foot pedals 22 while visually recognizing an affected area on the monitor 24. Thus, a command is input to the remote control apparatus 2. The command input to the remote control apparatus 2 is transmitted to the surgical robot 1.

### Configuration of Control System

As shown in FIG. 9, the robotic surgical system 100 includes a control device 130, an arm controller 31a, a positioner controller 31b, and operation controllers 110.

The control device 130 is accommodated in the medical cart 3 to communicate with the arm controller 31a and the positioner controller 31b, and controls the entire robotic surgical system 100. Specifically, the control device 130 communicates with and controls the arm controller 31a, the positioner controller 31b, and the operation controllers 110. The control device 130 is connected to the arm controller 31a, the positioner controller 31b, and the operation controllers 110 through a LAN, for example. The control device 130 is arranged inside the medical cart 3.

The arm controller 31a is arranged for each of the plurality of robot arms 60. That is, the same number of arm controllers 31a as the plurality of robot arms 60 are arranged inside the medical cart 3.

As shown in FIG. 9, the input 33 is connected to the control device 130 through a LAN, for example. The status indicator 53, the arm status indicator 54, the operation handle 34, the throttle 34a, the joystick 33b, the stabilizer 34c, and the electric cylinder 34d establish a serial communication connection with the positioner controller 31b through a wire line 145 by means of a communication network that allows information to be shared with each other. Although FIG. 9 shows that the status indicator 53, the arm status indicator 54, etc. are all connected to one wire line 145, in reality, the wire line 145 is arranged for each of the status indicator 53, the arm status indicator 54, the operation handle 34, the throttle 34a, the joystick 33b, the stabilizer 34c, and the electric cylinder 34d.

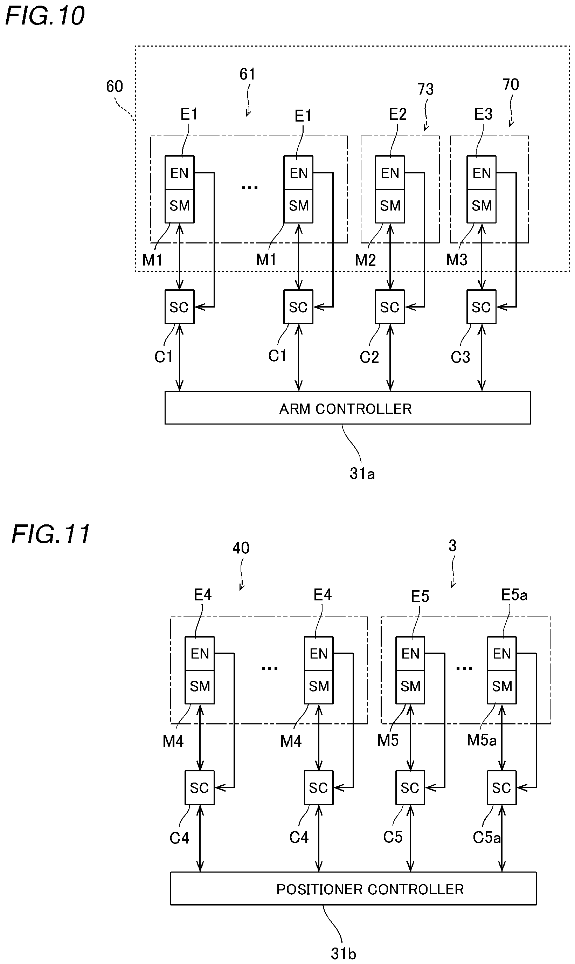

As shown in FIG. 10, the arm portion 61 includes a plurality of servomotors M1, encoders E1, and speed reducers so as to correspond to a plurality of joints 64. The encoders E1 detect rotation angles of the servomotors M1. The speed reducers slow down rotation of the servomotors M1 to increase the torques. Inside the medical cart 3, servo controllers C1 that control the servomotors M1 are provided adjacent to the arm controller 31a. The encoders E1 that detect the rotation angles of the servomotors M1 are electrically connected to the servo controllers C1.

Servomotors M2 to rotate driven members provided in the driven unit 4a of the surgical instrument 4, encoders E2, and speed reducers are arranged in the second link 73. The encoders E2 detect rotation angles of the servomotors M2. The speed reducers slow down rotation of the servomotors M2 to increase the torques. In the medical cart 3, servo controllers C2 are provided to control the servomotors M2 to drive the surgical instrument 4. The encoders E2 that detect the rotation angles of the servomotors M2 are electrically connected to the servo controllers C2. A plurality of servomotors M2, a plurality of encoders E2, and a plurality of servo controllers C2 are arranged.

The translation mechanism 70 includes a servomotor M3 to translationally move the surgical instrument 4, an encoder E3, and a speed reducer. The encoder E3 detects a rotation angle of the servomotor M3. The speed reducer slows down rotation of the servomotor M3 to increase the torque. In the medical cart 3, a servo controller C3 is provided to control the servomotor M3 to translationally move the surgical instrument 4. The encoder E3 that detects the rotation angle of the servomotor M3 is electrically connected to the servo controller C3.

As shown in FIG. 11, the positioner 40 includes a plurality of servomotors M4, encoders E4, and speed reducers so as to correspond to a plurality of joints 43 of the positioner 40. The encoders E4 detect the rotation angles of the servomotors M4. The speed reducers slow down rotation of the servomotors M4 to increase the torques.

The medical cart 3 includes front wheels as drive wheels and rear wheels that are steered by the operation handle 34. The rear wheels are arranged closer to the operation handle 34 than the front wheels. The medical cart 3 includes servomotors M5 to drive a plurality of front wheels of the medical cart 3, respectively, encoders E5, speed reducers, and brakes. The speed reducers slow down rotation of the servomotors M5 to increase the torques. Furthermore, a potentiometer P1 shown in FIG. 3 is provided in the operation handle 34 of the medical cart 3, and the servomotors M5 of the front wheels are driven based on a rotation angle detected by the potentiometer P1 according to the twist of the throttle 34a. The rear wheels of the medical cart 3 are of the dual wheel type, and the rear wheels are steered based on the rightward-leftward rotation of the operation handle 34. Furthermore, a potentiometer P2 shown in FIG. 3 is provided on a rotation shaft in the operation handle 34 of the medical cart 3, and servomotors M5a, encoders E5a, and speed reducers are provided on the rear wheels of the medical cart 3. The speed reducers slow down rotation of the servomotors M5a to increase the torques. The servomotors M5a are driven based on a rotation angle detected by the potentiometer P2 according to the rightward-leftward rotation of the operation handle 34. That is, steering of the rear wheels 3b by the rightward-leftward rotation of the operation handle 34 is power-assisted by the servomotors M5a.

The medical cart 3 is moved forward or rearward by driving the front wheels. Furthermore, the medical cart 3 is turned rightward or leftward by rotating the operation handle 34 of the medical cart 3 to steer the rear wheels.

As shown in FIG. 11, in the medical cart 3, servo controllers C4 are provided to control the servomotors M4 to move the positioner 40. The encoders E4 that detect the rotation angles of the servomotors M4 are electrically connected to the servo controllers C4. Furthermore, in the medical cart 3, servo controllers C5 are provided to control the servomotors M5 to drive the front wheels of the medical cart 3. The encoders E5 that detect the rotation angles of the servomotors M5 are electrically connected to the servo controllers C5. In the medical cart 3, servo controllers C5a are provided to control the servomotors M5a that power-assist the steering of the rear wheels of the medical cart 3. The encoders E5a that detect the rotation angles of the servomotors M5a are electrically connected to the servo controllers C5a.

As shown in FIG. 9, the control device 130 controls the robot arm 60 based on an operation received by the arm operation unit 80. For example, the control device 130 controls the robot arm 60 based on an operation received by the joystick 82 of the arm operation unit 80. Specifically, the arm controller 31a outputs an input signal input from the joystick 82 to the control device 130. The control device 130 generates position commands based on the received input signal and the rotation angles detected by the encoders E1, and outputs the position commands to the servo controllers C1 via the arm controller 31a. The servo controllers C1 generate current commands based on the position commands input from the arm controller 31a and the rotation angles detected by the encoders E1, and output the current commands to the servomotors M1. Thus, the robot arm 60 is moved according to an operation command input to the joystick 82.

The control device 130 controls the robot arm 60 based on an input signal from either linear switch 83 of the arm operation unit 80. Specifically, the arm controller 31a outputs the input signal input from the linear switch 83 to the control device 130. The control device 130 generates a position command(s) based on the received input signal and the rotation angle(s) detected by the encoders E1 or the encoder E3, and outputs the position command(s) to the servo controllers C1 or the servo controller C3 via the arm controller 31a. The servo controllers C1 or the servo controller C3 generates a current command(s) based on the position command(s) input from the arm controller 31a and the rotation angle(s) detected by the encoders E1 or the encoder E3, and outputs the current command(s) to the servomotors M1 or the servomotor M3. Thus, the robot arm 60 is moved according to an operation command input to the linear switch 83.

The positioner controller 31b is arranged in the medical cart 3. The positioner controller 31b controls the positioner 40 and the medical cart 3. Servomotors SM, encoders EN, and speed reducers are provided in the positioner 40 so as to correspond to the plurality of joints 43 of the positioner 40. Servo controllers SC are provided in the medical cart 3 to control the servomotors SM of the positioner 40. Servomotors SM that drive the plurality of front wheels of the medical cart 3, encoders EN, speed reducers, servo controllers SC, and brakes are provided in the medical cart 3.

The operation controllers 110 are arranged in a main body of the remote control apparatus 2. The operation controllers 110 control the operation unit 120. The operation controllers 110 are provided so as to correspond to the operation unit 120L and the operation unit 120R, respectively. Servomotors SM, encoders EN, and speed reducers are provided in the operation unit 120 so as to correspond to a plurality of joints of the operation unit 120. Servo controllers SC that control the servomotors SM of the operation unit 120 are provided adjacent to the operation controllers 110 in the main body of the remote control apparatus 2.

### Control Method for Robotic Surgical System

A control method for the robotic surgical system 100 is now described. Four trocars T4 are arranged in advance on the body surface S of the patient P placed on a surgical table 5. The surgical instruments 4 are not attached to the robot arms 60.

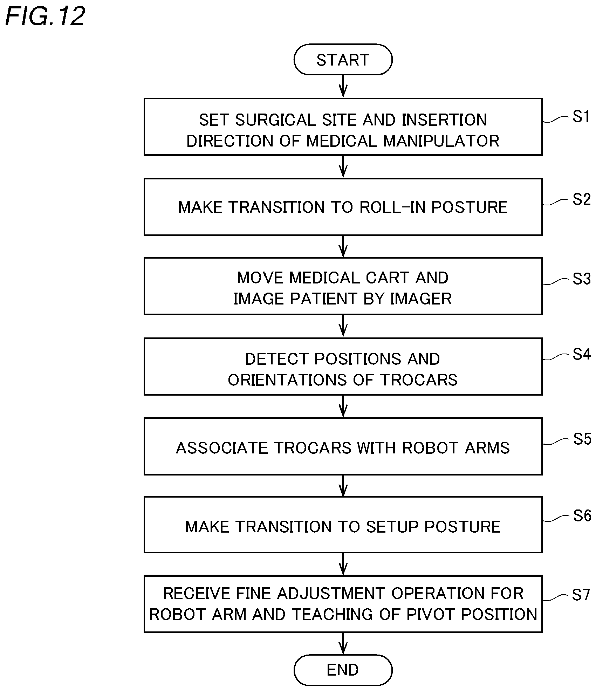

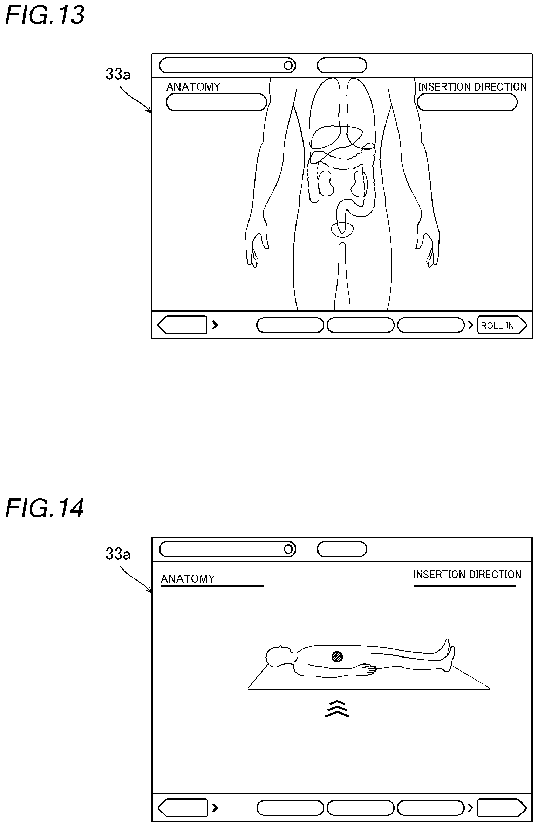

As shown in FIG. 12, in step S1, preparations for positioning the surgical robot 1 are performed. Specifically, as shown in FIG. 13, on a touch panel of the display 33a, a surgical site such as an abdomen and a direction in which the surgical robot 1 is inserted into the patient P are selected.

Then, in step S2, as shown in FIG. 13, a roll-in button displayed on the display 33a is pressed. Thus, a roll-in mode is set, and the movement operation of the arm base 50 and the robot arms 60 is controlled such that the surgical robot 1 takes a roll-in posture. The roll-in posture refers to a posture in which each robot arm 60 is folded so as not to interfere with the patient P when the robot arm 60 is positioned above the patient P by movement of the surgical robot 1, in which the arm base 50 is arranged by the positioner 40 such that the imager 51 provided on the arm base 50 can image a region vertically therebelow, and in which the arm base 50 is arranged by the positioner 40 such that the arrangement direction of each robot arm 60 corresponds to the surgical site and the insertion direction based on information on the surgical site selected in step S1 and information on the insertion direction selected in step S1. That is, after the roll-in button displayed on the display 33a is pressed such that a transition to the roll-in mode is made, when the joystick 33b is operated while the enable switch 33c is being pressed to enable movement of the positioner 40, the control device 130 moves the positioner 40 and each robot arm 60 such that the surgical robot 1 automatically takes the roll-in posture.

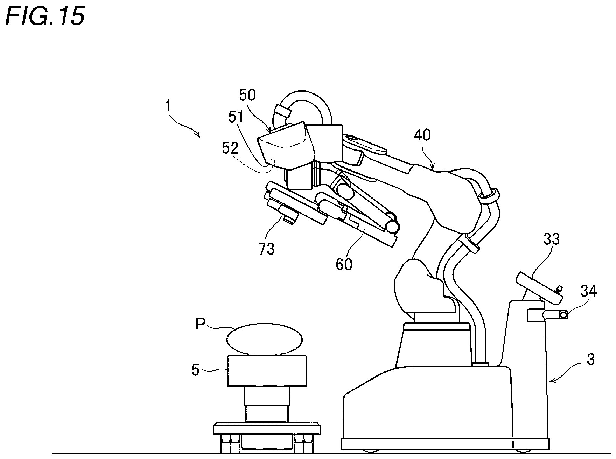

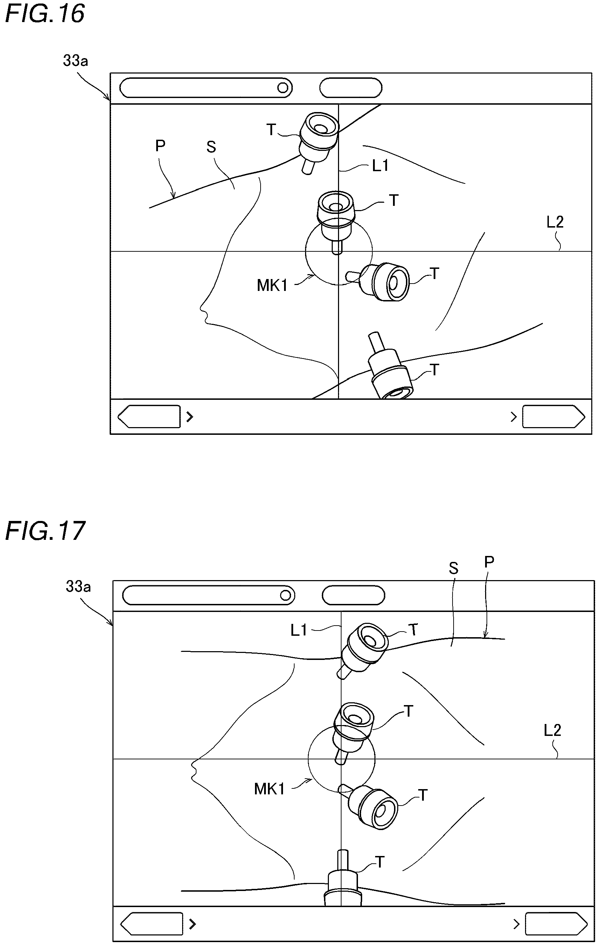

Then, in step S3, as shown in FIG. 14, after the movement operation of the robot arm 60, a screen of the display 33a is switched to an image captured by the imager 51. Then, as shown in FIG. 15, the medical cart 3 is moved closer to the surgical table 5, and the imager 51 provided on the arm base 50 images the surgical table 5 and the patient P placed on the surgical table 5. Specifically, the operator such as a nurse or a technician operates the operation handle 34 while viewing the image displayed on the display 33a to move the medical cart 3 to the vicinity of the patient P. Thus, the trocars T are placed directly below the imager 51. Then, as shown in FIG. 16, the trocar T is placed inside a substantially circular first mark MK1 on the display 33a. Furthermore, as shown in FIG. 17, the operator operates the positioner 40 using the joystick 33b such that the remaining trocars T are placed on a first line L1 or a second line L2 of a second mark MK2 while one trocar T is placed inside the substantially circular first mark MK1 on the display 33a.

Then, in step S4, the control device 130 detects the positions and orientations of the trocars T based on at least the image of the patient P captured by the imager 51 after the medical cart 3, the positioner 40, the arm base 50, and the robot arms 60 are moved toward the patient P by the operator based on the image captured by the imager 51. Specifically, the control device 130 detects the two-dimensional coordinates of the trocars T in a horizontal plane based on the image of the patient P captured by the imager 51. The control device 130 detects distances to the trocars T along a vertical direction based on the detection results of the distance sensor 52. Thus, the control device 130 acquires the three-dimensional coordinates of the trocars T. Furthermore, the orientations of the trocars T are acquired based on the image of the patient P captured by the imager 51. The orientations of the trocars T refer to the longitudinal orientations of the trocars T, for example. The control device 130 detects the positions and orientations of all four trocars T. Furthermore, the medical cart 3, the positioner 40, the arm base 50, and the robot arms 60 are examples of a robot main body.

Then, in step S5, the control device 130 associates the four trocars T with the four robot arms 60. That is, the control device 130 determines into which of the four trocars T the surgical instrument 4 attached to which of the robot arms 60 is to be inserted.

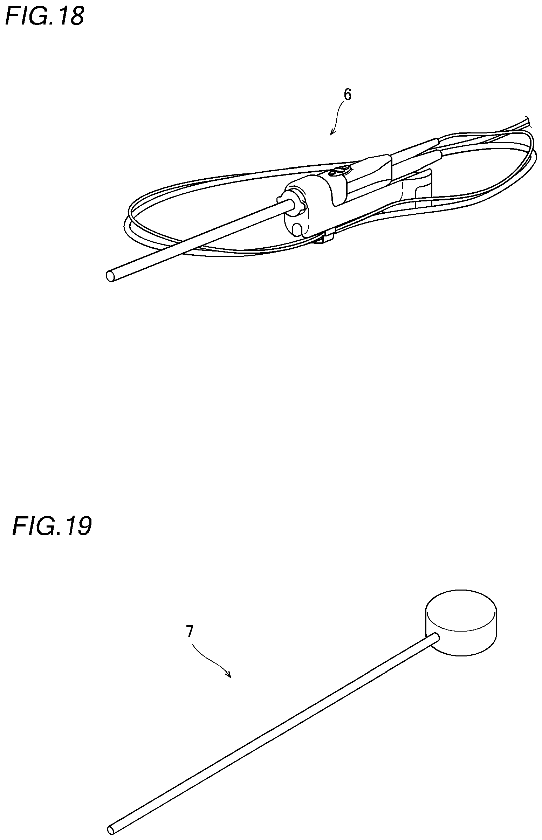

Then, in step S6, the control device 130 moves the plurality of robot arms 60 to the setup postures. The setup posture is different from the roll-in posture in which each robot arm 60 is folded, and refers to a posture in which a distance between the robot arms 60 is widened such that the endoscope 6 shown in FIG. 18 or the pivot position setting instrument 7 shown in FIG. 19 can be easily attached to each of the plurality of robot arms 60. The pivot position setting instrument 7 is an instrument that is attached to the distal end of the robot arm 60 to which the instrument is to be attached, instead of the instrument, when the pivot position PP is set.

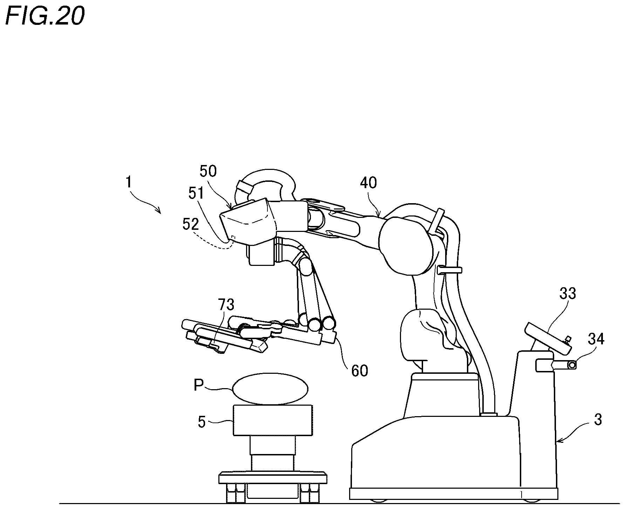

In the present embodiment, as shown in FIG. 20, the control device 130 moves the second link 73 toward the trocar T based on the detected position and orientation of the trocar T. Specifically, the control device 130 moves the second link 73 toward the trocar T by moving the robot arm 60.

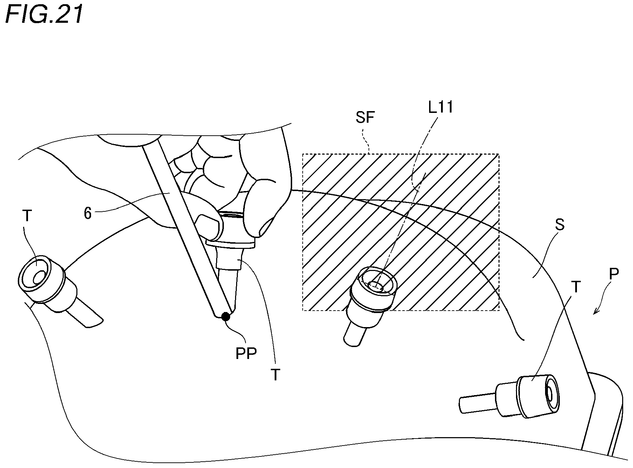

In the present embodiment, as shown in FIG. 21, the control device 130 moves the second link 73 such that the shaft 4c of the surgical instrument 4 is placed in a plane SF along the vertical direction including an axis L11 of the trocar T based on the detected position and orientation of the trocar T. That is, the control device 130 moves the second link 73 such that the shaft 4c of the surgical instrument 4 is placed in the plane SF that includes the axis L11 and is perpendicular to the body surface S of the patient P.

In the present embodiment, the control device 130 moves the second link 73 to a position for attaching the endoscope 6 or the pivot position setting instrument 7 to the second link 73 in order to set the pivot position PP based on the detected position and orientation of the trocar T. That is, the control device 130 moves the second link 73 to the vicinity of the trocar T. The pivot position PP refers to a position that serves as a fulcrum for movement of the surgical instrument 4 attached to the robot arm 60. Furthermore, the position for attaching the endoscope 6 or the pivot position setting instrument 7 refers, for example, to a position at which the endoscope 6 or the pivot position setting instrument 7 does not contact the patient P even when the endoscope 6 or the pivot position setting instrument 7 is attached to the robot arm 60 and at which a distal end of the endoscope 6 or the pivot position setting instrument 7 is located in the vicinity of the body surface S of the patient P.

In the present embodiment, the control device 130 moves the second link 73 of the robot arm 60c to which the endoscope 6 is to be attached and the second links 73 of the robot arms 60a, 60b, and 60d to which the surgical instruments 4 other than the endoscope 6 are to be attached toward the trocars T based on the detected positions and orientations of the trocars T. Each robot arm 60 is moved toward the trocar T all at once, for example. Each robot arm 60 may be moved sequentially. After each robot arm 60 is moved, the operator attaches the endoscope 6 or the pivot position setting instrument 7 to each of the plurality of robot arms 60.

Then, in step S7, in the present embodiment, the control device 130 receives a fine adjustment operation for the position of the robot arm 60 with the endoscope 6 or the pivot position setting instrument 7 attached to the second link 73. Fine adjustment of the position of the robot arm 60 is performed by the operator operating the joystick 82 or the linear switch 83. After receiving the fine adjustment operation for the position of the robot arm 60, the control device 130 receives an instruction to set the pivot position PP. Specifically, as shown in FIG. 21, when the pivot button 85 is pressed in a state in which the distal end of the endoscope 6 or the pivot position setting instrument 7 attached to the distal end side of the robot arm 60 has been moved to a position corresponding to the insertion position of the trocar T inserted into the body surface S of the patient P by operating the robot arm 60 using the arm operation unit 80, the control device 130 causes the storage 32 to store the pivot position PP. The pivot position PP is stored as one coordinate point, and in pivot position PP setting, the direction of the surgical instrument 4 is not set.

### Advantages of Present Embodiment

The control device 130 is configured or programmed to detect the position and orientation of the trocar T based on the detection results of the imager 51 and the distance sensor 52, and move the second link 73 toward the trocar T based on the detected position and orientation of the trocar T. Accordingly, the second link 73 of the robot arm 60 is automatically moved to an appropriate position based on the detected position and orientation of the trocar T, and thus the operator does not need to manually move the second link 73 of the robot arm 60. Therefore, the workload on the operator to place the robot arm 60 at the appropriate position can be reduced. Furthermore, the robot arm 60 is automatically placed at the appropriate position, and thus the time required to place the robot arm 60 at the appropriate position can be reduced as compared with a case in which the robot arm 60 is manually placed at the appropriate position.

The control device 130 is configured or programmed to detect the position and orientation of the trocar T based on the image captured by the imager 51 and the distance to the patient P detected by the distance sensor 52. Accordingly, the position and orientation of the trocar T can be easily detected based on the image captured by the imager 51 and the distance to the patient P detected by the distance sensor 52.

According to the present embodiment, the control device 130 is configured or programmed to move the second link 73 such that the shaft 4c of the surgical instrument 4 is placed in the plane SF along the vertical direction including the axis L11 of the trocar T based on the detected position and orientation of the trocar T. Accordingly, the direction of the shaft 4c of the surgical instrument 4 can be aligned with the orientation of the trocar T.

The control device 130 is configured or programmed to detect the position and orientation of the trocar T based on at least the image of the patient P captured by the imager 51 after the medical cart 3 is moved toward the patient P based on the image captured by the imager 51, and move the second link 73 toward the trocar T based on the detected position and orientation of the trocar T. Accordingly, the operation to move the medical cart 3 toward the patient P and the operation to move the second link 73 toward the trocar T are performed in succession, and thus the time required for the operation to place the robot arm 60 at the appropriate position can be further reduced.

The control device 130 is configured or programmed to move the second link 73 to the position for attaching the surgical instrument 4 or the pivot position setting instrument 7 to the second link 73 in order to set the pivot position PP based on the detected position and orientation of the trocar T. Accordingly, the workload on the operator to place the robot arm 60 at the appropriate position in order to set the pivot position PP can be reduced.

The control device 130 is configured or programmed to move, toward the trocar T, the second link 73 with no surgical instrument 4 attached thereto, and receive an instruction to set the pivot position PP after receiving a fine adjustment operation for the position of the robot arm 60 with the surgical instrument 4 or the pivot position setting instrument 7 attached to the second link 73. Accordingly, the control device 130 receives the fine adjustment operation for the position of the robot arm 60, and thus the operator can set the pivot position PP at an appropriate position.

The imager 51 and the distance sensor 52 are arranged on the arm base 50. Accordingly, the imager 51 and the distance sensor 52 move together with the robot arm 60, and thus the robot arm 60 can be easily aligned with the position and orientation of the trocar T detected by the imager 51 and the distance sensor 52.

The distance sensor 52 is arranged adjacent to the imager 51 on the arm base 50. Accordingly, a distance between the imager 51 and the distance sensor 52 is reduced, and thus the accuracy of detecting the position and orientation of the trocar T can be improved.

The control device 130 is configured or programmed to move the second link 73 of the robot arm 60c to which the endoscope 6 is to be attached and the second links 73 of the robot arms 60a, 60b, and 60d to which the surgical instruments 4 other than the endoscope 6 are to be attached toward the trocars T based on the detected positions and orientations of the trocars T. Accordingly, even when a plurality of robot arms 60 are arranged, the workload on the operator to place the plurality of robot arms 60 at appropriate positions can be reduced.

### Modified Examples

The embodiment disclosed this time must be considered as illustrative in all points and not restrictive. The scope of the present disclosure is not shown by the above description of the embodiment but by the scope of claims for patent, and all modifications or modified examples within the meaning and scope equivalent to the scope of claims for patent are further included.

While the example in which the imager 51 and the distance sensor 52 are used as detectors to detect the position and orientation of the trocar T inserted into the patient P has been shown in the aforementioned embodiment, the present disclosure is not limited to this. For example, the position and orientation of the trocar T may be detected by a device other than the imager 51 and the distance sensor 52.

While the example in which a TOF sensor is used as the distance sensor 52 has been shown in the aforementioned embodiment, the present disclosure is not limited to this. For example, as the distance sensor 52, an optical (lidar: light detection and ranging) sensor, a radio wave (radar) sensor, or an ultrasonic sensor may be used, or a distance sensor using a phase difference detection method or a triangulation method may be used.

While the example in which both the imager 51 and the distance sensor 52 are used has been shown in the aforementioned embodiment, the present disclosure is not limited to this. For example, a stereo camera may be used as the imager 51, and the position and orientation of the trocar T may be detected based on the detection results of the stereo camera. Thus, the position and orientation of the trocar T can be detected only by the imager 51 without providing the distance sensor 52.

While the example in which the imager 51 and the distance sensor 52 are arranged on the arm base 50 has been shown in the aforementioned embodiment, the present disclosure is not limited to this. For example, the imager 51 and the distance sensor 52 may be arranged at a location other than the arm base 50.

While the example in which the imager 51 and the distance sensor 52 are arranged adjacent to each other on the arm base 50 has been shown in the aforementioned embodiment, the present disclosure is not limited to this. For example, the imager 51 and the distance sensor 52 may be arranged far apart from each other on the arm base 50.

While the example in which the control device 130 is arranged inside the medical cart 3 has been shown in the aforementioned embodiment, the present disclosure is not limited to this. For example, the control device 130 may be arranged outside the medical cart 3.

While the example in which the control device moves the second link 73 such that the shaft 4c of the surgical instrument 4 is placed in the plane SF along the vertical direction including the axis L11 of the trocar T based on the detected position and orientation of the trocar T has been shown in the aforementioned embodiment, the present disclosure is not limited to this. For example, the control device 130 may set the axis L11 of the trocar T based on the detected position and orientation of the trocar T, and move the second link 73 such that the shaft 4c of the surgical instrument 4 is placed on the axis L11 of the trocar T. Thus, the direction of the shaft 4c of the surgical instrument 4 can be more accurately aligned with the orientation of the trocar T.

While the example in which four robot arms 60 are provided has been shown in the aforementioned embodiment, the present disclosure is not limited to this. In the present disclosure, the number of robot arms 60 may be any other number as long as at least one robot arm is provided.

While the example in which each of the arm portion 61 and the positioner 40 includes a 7-axis articulated robot has been shown in the aforementioned embodiment, the present disclosure is not limited to this. For example, each of the arm portion 61 and the positioner 40 may include an articulated robot having an axis configuration other than the 7-axis articulated robot. The axis configuration other than the 7-axis articulated robot includes six axes or eight axes, for example.

While the example in which the surgical robot 1 includes the medical cart 3, the positioner 40, and the arm base 50 has been shown in the aforementioned embodiment, the present disclosure is not limited to this. For example, the surgical robot 1 may not include the medical cart 3, the positioner 40, or the arm base 50, but may include only the robot arms 60.

The functionality of the elements disclosed herein may be implemented using circuitry or processing circuitry that includes general purpose processors, special purpose processors, integrated circuits, application specific integrated circuits (ASICs), conventional circuitry and/or combinations thereof that are configured or programmed to perform the disclosed functionality. Processors are considered processing circuitry or circuitry as they include transistors and other circuitry therein. In the present disclosure, the circuitry, units, or means are hardware that carries out the recited functionality or hardware that is programmed to perform the recited functionality. The hardware may be hardware disclosed herein or other known hardware that is programmed or configured to carry out the recited functionality. When the hardware is a processor that may be considered a type of circuitry, the circuitry, means, or units are a combination of hardware and software, and the software is used to configure the hardware and/or processor.

### Aspects

It will be appreciated by those skilled in the art that the exemplary embodiments described above are specific examples of the following aspects.

### (Item 1)

A robotic surgical system comprising:
a robot arm including a surgical instrument mount to which a surgical instrument is attached;
a detector to detect a position and orientation of a trocar inserted into a patient; and
a control device; wherein
the control device is configured or programmed to:
   detect the position and orientation of the trocar based on a detection result of the detector; and
   move the surgical instrument mount toward the trocar based on the detected position and orientation of the trocar.

### (Item 2)

The robotic surgical system according to item 1, wherein
the detector includes at least an imager to image the patient of the imager and a distance sensor to detect a distance to the patient; and
the control device is configured or programmed to detect the position and orientation of the trocar based on at least an image of the patient captured by the imager.

### (Item 3)

The robotic surgical system according to item 1 or 2, wherein
the surgical instrument further includes a shaft; and
the control device is configured or programmed to move the surgical instrument mount such that the shaft of the surgical instrument is placed in a plane along a vertical direction including an axis of the trocar based on the detected position and orientation of the trocar.

### (Item 4)

The robotic surgical system according to item 3, wherein the control device is configured or programmed to:
set the axis of the trocar based on the detected position and orientation of the trocar; and
move the surgical instrument mount such that the shaft of the surgical instrument is placed on the axis of the trocar.

### (Item 5)

The robotic surgical system according to any one of items 1 to 4, further comprising:
a robot main body; wherein
the detector includes at least an imager to image the patient of the imager and a distance sensor to detect a distance to the patient; and
the control device is configured or programmed to:
   detect the position and orientation of the trocar based on at least an image of the patient captured by the imager after the robot main body is moved toward the patient based on an image captured by the imager; and
   move the surgical instrument mount toward the trocar based on the detected position and orientation of the trocar.

### (Item 6)

The robotic surgical system according to any one of items 1 to 5, wherein the control device is configured or programmed to move the surgical instrument mount to a position for attaching the surgical instrument or a pivot position setting instrument to the surgical instrument mount in order to set a pivot position that serves as a fulcrum for movement of the surgical instrument attached to the robot arm based on the detected position and orientation of the trocar.

### (Item 7)

The robotic surgical system according to item 6, wherein the control device is configured or programmed to:
move, toward the trocar, the surgical instrument mount with no surgical instrument attached thereto; and
receive an instruction to set the pivot position after receiving a fine adjustment operation for a position of the robot arm with the surgical instrument or the pivot position setting instrument attached to the surgical instrument mount.

### (Item 8)

The robotic surgical system according to any one of items 1 to 7, further comprising:
an arm base to which the robot arm is attached; wherein
the detector is arranged on the arm base.

### (Item 9)

The robotic surgical system according to item 8, wherein
the detector includes an imager to image the patient and a distance sensor to detect a distance to the patient;
the imager is arranged on the arm base; and
the distance sensor is arranged adjacent to the imager on the arm base.

### (Item 10)

The robotic surgical system according to any one of items 1 to 9, wherein
the robot arm is a first robot arm;
the robotic surgical system further comprises a second robot arm to which an endoscope is attached; and
the control device is configured or programmed to move the surgical instrument mount of each of the first robot arm and the second robot arm toward the trocar based on the detected position and orientation of the trocar.

### (Item 11)

A surgical robot comprising:
a robot arm including a surgical instrument mount to which a surgical instrument is attached;
a detector to detect a position and orientation of a trocar inserted into a patient; and
a control device; wherein
the control device is configured or programmed to:
   detect the position and orientation of the trocar based on a detection result of the detector; and
   move the surgical instrument mount toward the trocar based on the detected position and orientation of the trocar.

### (Item 12)

A control method for a robotic surgical system, the robotic surgical system including a robot arm including a surgical instrument mount to which a surgical instrument is attached, a detector to detect a position and orientation of a trocar inserted into a patient, and a control device, the control method comprising:
detecting the position and orientation of the trocar based on a detection result of the detector; and
moving the surgical instrument mount toward the trocar based on the detected position and orientation of the trocar.

## Claims

1. A robotic surgical system comprising:
a robot arm including a surgical instrument mount to which a surgical instrument is attached;
a detector to detect a position and orientation of a trocar inserted into a patient; and
a control device; wherein
the control device is configured or programmed to:
detect the position and orientation of the trocar based on a detection result of the detector; and
move the surgical instrument mount toward the trocar based on the detected position and orientation of the trocar.

2. The robotic surgical system according to claim 1, wherein
the detector includes at least an imager to image the patient of the imager and a distance sensor to detect a distance to the patient; and
the control device is configured or programmed to detect the position and orientation of the trocar based on at least an image of the patient captured by the imager.

3. The robotic surgical system according to claim 1, wherein
the surgical instrument further includes a shaft; and
the control device is configured or programmed to move the surgical instrument mount such that the shaft of the surgical instrument is placed in a plane along a vertical direction including an axis of the trocar based on the detected position and orientation of the trocar.

4. The robotic surgical system according to claim 3, wherein the control device is configured or programmed to:
set the axis of the trocar based on the detected position and orientation of the trocar; and
move the surgical instrument mount such that the shaft of the surgical instrument is placed on the axis of the trocar.

5. The robotic surgical system according to claim 1, further comprising:
a robot main body; wherein
the detector includes at least an imager to image the patient of the imager and a distance sensor to detect a distance to the patient; and
the control device is configured or programmed to:
detect the position and orientation of the trocar based on at least an image of the patient captured by the imager after the robot main body is moved toward the patient based on an image captured by the imager; and
move the surgical instrument mount toward the trocar based on the detected position and orientation of the trocar.

6. The robotic surgical system according to claim 1, wherein the control device is configured or programmed to move the surgical instrument mount to a position for attaching the surgical instrument or a pivot position setting instrument to the surgical instrument mount in order to set a pivot position that serves as a fulcrum for movement of the surgical instrument attached to the robot arm based on the detected position and orientation of the trocar.

7. The robotic surgical system according to claim 6, wherein the control device is configured or programmed to:
move, toward the trocar, the surgical instrument mount with no surgical instrument attached thereto; and
receive an instruction to set the pivot position after receiving a fine adjustment operation for a position of the robot arm with the surgical instrument or the pivot position setting instrument attached to the surgical instrument mount.

8. The robotic surgical system according to claim 1, further comprising:
an arm base to which the robot arm is attached; wherein
the detector is arranged on the arm base.

9. The robotic surgical system according to claim 8, wherein
the detector includes an imager to image the patient and a distance sensor to detect a distance to the patient;
the imager is arranged on the arm base; and
the distance sensor is arranged adjacent to the imager on the arm base.

10. The robotic surgical system according to claim 1, wherein
the robot arm is a first robot arm;
the robotic surgical system further comprises a second robot arm to which an endoscope is attached; and
the control device is configured or programmed to move the surgical instrument mount of each of the first robot arm and the second robot arm toward the trocar based on the detected position and orientation of the trocar.

11. A surgical robot comprising:
a robot arm including a surgical instrument mount to which a surgical instrument is attached;
a detector to detect a position and orientation of a trocar inserted into a patient; and
a control device; wherein
the control device is configured or programmed to:
detect the position and orientation of the trocar based on a detection result of the detector; and
move the surgical instrument mount toward the trocar based on the detected position and orientation of the trocar.

12. A control method for a robotic surgical system, the robotic surgical system including a robot arm including a surgical instrument mount to which a surgical instrument is attached, a detector to detect a position and orientation of a trocar inserted into a patient, and a control device, the control method comprising:
detecting the position and orientation of the trocar based on a detection result of the detector; and
moving the surgical instrument mount toward the trocar based on the detected position and orientation of the trocar.
